# EUROPEAN PATENT SPECIFICATION

(11) **EP 2 579 770 B1**
(45) Date of publication and mention of the grant of the patent: **13.09.2017**
(21) Application number: 11725080.3
(22) Date of filing: 13.06.2011
(51) Int. Cl.: A61B 5/00, A61B 5/0408

(54) **ELECTRODE FOR ELECTROCARDIOGRAPH AND CHEST BELT THEREFOR**
ELEKTRODE FÜR EINEN ELEKTROKARDIOGRAF UND THORAXGURT DAFÜR
ÉLECTRODE POUR ÉLECTROCARDIOGRAPHE ET CEINTURE THORACIQUE ASSOCIÉE

(30) Priority: 14.06.2010 IT MI20101071
(43) Date of publication of application: 17.04.2013
(73) Proprietor: Marcolongo, Alberto, 20015 Parabiago MI (IT)
(72) Inventor: Marcolongo, Alberto, 20015 Parabiago MI (IT)
(74) Representative: Spina, Alessandro
(86) International application number: PCT/EP2011/059759
(87) International publication number: WO 2011/157669

(56) References cited:
- EP-A2- 1 946 698
- US-A- 3 380 445
- US-A- 5 891 045
- US-A- 6 117 077
- US-A1- 2004 210 158
- US-A1- 2005 154 438
- US-A1- 2008 177 168
- US-A1- 2009 076 364
- US-A1- 2010 022 865
- US-B1- 6 456 872

## Description

The present invention relates to an electrocardiograph electrode and to a chest belt suitable to allow its contact with the body of a patient.

It is known that the measurement of heart electric activity is based on a physiological principle. The onset of electric impulses in the myocardium in fact causes potential differences that vary over space and time and may be registered by way of a plurality of electrodes placed in contact with the body of a patient at predetermined locations. In order to improve the contact characteristics, the conductive surface of the electrodes is covered with special ion gels suitable to decrease the skin-electrode contact impedance. Moreover, the skin-electrode contact is further improved by means of specific belts, such as the well-known chest belts, or by using suction cups, patches and the like.

Examples of electrocardiograph electrodes and related contact devices are disclosed, for example, in patent publications US 2008/177168, US 6117077, US 3380445, US 6456872, US 2005/154438 and EP 1946698.

Potential differences can be recorded by the electrodes thanks to the conductivity of the tissue fluids of the human body. The electrocardiogram chart is a very effective means to monitor heart electric activity and diagnose possible diseases of mechanical or bioelectric nature.

It is also known that, during the recording of an electrocardiogram, the electric signals acquired by the electrodes are affected by noise. Noise can be of an electromagnetic nature, e.g. relating to the operating frequency of the electric network of the place where an electrocardiograph is installed and/or other electronic instruments arranged proximate thereto.

Noise can also be caused by contact problems e.g. deriving from a poor amount of ion gel on individual electrodes, a low tensioning of the belts, chest belts in particular, used to press the electrodes against the patient's body, the presence of foreign bodies, e.g. metal micro-particles, between electrodes and skin, and also from involuntary movements, e.g. tremors, of the patient, which may temporarily cause the detachment of an electrode from the patient's body. These problems are even more serious when carrying out ergometric tests, wherein an electrocardiogram is recorded during the execution of a physical effort, i.e. under continuous movement conditions.

Electromagnetic noise can be eliminated by way of suitable filters well known to those skilled in the art, which are mounted on the electrodes wires and/or directly on the data acquisition instrumentation.

Noise deriving from contact problems between individual electrodes and patient's body can be addressed by using special electrodes as those described in patent US 5891045. According to this patent, each electrode may be configured as a multi-segment electrode wherein each segment is provided with a pad made of a conductive material and has a certain degree of flexibility. In this case an electrocardiogram is generated by processing and combining the analog signals recorded from the individual pads formed on the flexible segments, thus allowing to minimize noise problems. However, despite the deformable configuration of the segments of the electrodes, contact problems may still occur, in particular in the case of ergometric tests.

Therefore, there is still the need to further improve the contact characteristics, in particular in relation with ergometric tests, which is an object of the present invention.

An idea of solution underlying the invention is to provide the electrode with a plurality of contact pads arranged on one or more contact members and suitable to establish an electric contact with the patient's skin, and to restrain the contact members and the contact pads to a supporting member by way of elastic members.

As a result, it is possible to exploit the advantages of a multi-pad electrode configuration in terms of data acquisition, while ensuring a consistent contact between electrodes and skin when the patient moves, as typically occurs during ergometric tests.

The contact pads of the electrodes may advantageously be covered by a layer of a ion gel suitable to decrease the contact impedance with the patient's skin. The overall shape of the contact pads covered by the layer of ion gel is preferably convex, thus allowing to achieve a more uniform contact with the patient's skin that under contact pressure is inevitably deformed in a plurality of locations corresponding to the skin-electrode pads contact points forming concave depressions.

The electrode according to the invention may be advantageously integrated into a chest belt, allowing to effectively press it against the patient's body.

The chest belt may advantageously include several electrodes arranged according to a predefined pattern, such as the standard twelve-lead pattern typically used for recording an electrocardiogram. The use of a chest belt supporting a plurality of electrodes according to the invention is also advantageous because the chest belt offers a wide area for accommodating and/or housing the electric wires connected to the contact pads.

The chest belt may also advantageously comprise one or more wireless transmitters connected to the electrodes and suitable to transmit in real time the signals acquired from each electrode to a remote control unit. It is thus possible to completely eliminate the wires typically used in electrocardiographs for connecting the electrodes to the remote control unit, thereby achieving significant benefits in terms of overall size, ease of installation and time of execution of an electrocardiogram. The invention is defined by the independent claims. Further advantages and features of the electrode and the chest belt according to the present invention will be clear to those skilled in the art from the following detailed and non-limiting description of embodiments thereof with reference to the attached drawings in which:
- Figure 1 is a top view of an electrocardiograph electrode according to the present invention;
- Figure 2 is a bottom view of the electrode of figure 1;
- Figure 3 is a cross-sectional view taken along line III-III of figure 1;
- Figures 4 and 5 show alternative electrode embodiments of the electrode of figure 1;
- Figure 6 shows a chest belt comprising a plurality of electrodes according to the invention; and
- Figure 7 shows an alternative embodiment of the chest belt of figure 6.

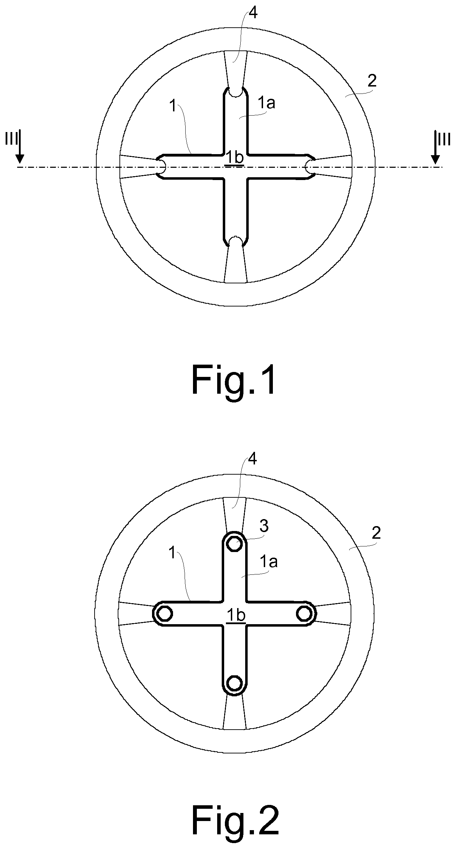

Referring to figures 1 to 3, the electrocardiograph electrode according to the invention includes a contact member 1 restrained to a supporting member 2, for example ring-shaped, suitable to allow to press the contact member 1 against the body of a patient by means of a chest belt, a patch and the like.

The contact member 1 comprises a plurality of contact pads 3 suitable to establish an electric contact with the patient's body. The contact pads 3 may be made of materials such as silver, gold or platinum, well-known to those skilled in the art for their characteristics of electric conductivity. The contact pads 3 are preferably completely covered by layer of a ion gel (not shown) suitable to decrease the contact impedance with the patient's skin. The overall shape of the contact pads 3 covered by the layer of ion gel is preferably convex, which advantageously allows to establish optimal contact conditions with the patient's body and to minimize the contact problems caused by the concave local depressions that are formed in the skin as a result of the pressure exerted by the contact member 1. The convex shape of the contact pads 3 with their ion gel layer in fact matches the concave shape of the depressions formed on the skin by the contact pads 3 themselves.

In the embodiment shown in figures 1 to 3, the contact member 1 comprises a body provided with a plurality of arms 1a which protrude radially outwards from a central portion 1b. The contact pads 3 are respectively placed at the end of each arm 1a and the contact member 1 is connected to the supporting member 2 at the ends of the arms 1a. Alternatively, the contact member 1 may be connected to the supporting member 2 at the central portion 1b.

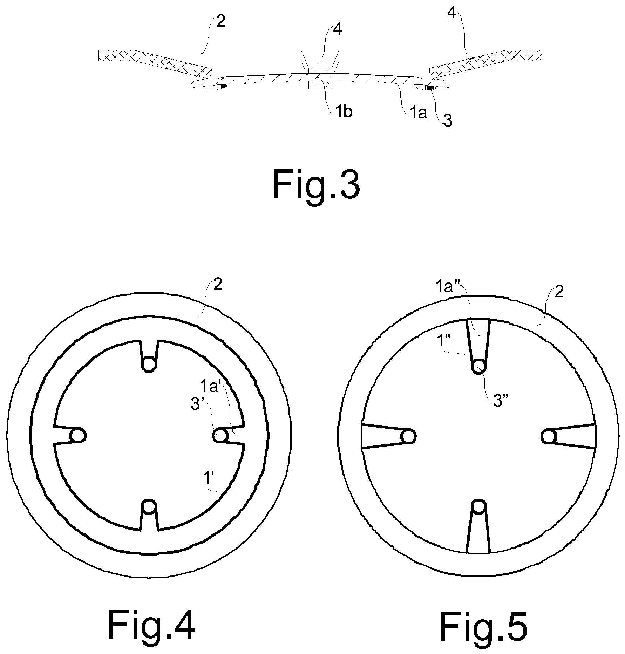

As shown in the cross-section of figure 3, the contact member 1 is concave-shaped and its cavity is so arranged to face the patient's body in an operating condition of the electrode. Thus only the contact pads 3, that are placed at the ends of the arms 1a, will contact the patient's body. The arms 1a may be curved or simply bent with respect to the central portion 1b.

The contact member 1 and consequently the contact pads 3 are restrained to the supporting member 2 by way of a plurality of elastic members 4. The elastic members 4 are preferably in the form of cantilevers and may be integrally manufactured with either the supporting member 2 or the contact member 1, or they may be made as separate components mounted between the supporting member 2 and contact member 1.

The use of the elastic members 4 provides a high degree of deformability to the electrode of the invention, thus ensuring a consistent contact between the contact pads 3 and the patient's skin in all movement conditions, in particular during ergometric tests.

Still referring to figure 3, the elastic members 4 are preferably arranged at an angle with respect to the supporting member 2, so as to protrude towards the patient's body in an operative condition. This allows to exert a pressure against the patient's body in an operating condition, thus improving the contact between skin and pads 3.

The contact member 1 may advantageously be removably mounted on the supporting member 2, e.g. press fitted, snap fitted and the like. It is thus possible to repair and/or service the electrode according to the invention by simply replacing the contact member 1, when this can no longer ensure the desired contact performances due to soiling, permanent mechanical deformations and the like. This configuration is particularly advantageous when the electrode is used together with other electrodes in an electrocardiograph chest belt. The possibility of removing the contact members of the electrodes, i.e. only the part of each electrode intended to contact the skin of a patient, allows to leave their supporting structures and the related electric wires in place while mounting new contact members for a new patient. In other words, the preparation of the chest belt for a new patient becomes quicker, easier and cheaper.

Figures 4 and 5 show two alternative embodiments of the contact member 1.

Figure 4 shows in particular a contact member 1' comprising a ring-shaped body from which a plurality of elastic members 4' in the form of cantilevers protrude radially inwards. The contact pads 3' are arranged at the ends of the elastic members 4'. The contact member 1' is restrained to the supporting member 2 in a known way, e.g. by way of a snap connection, thus forming a rigid body therewith. Similarly to the above-described embodiment, the elastic members 4' may be arranged at an angle with respect to the supporting member 2, so as to protrude towards the patient's body in an operative condition in order to increase the contact pressure.

This embodiment has the advantage of providing the contact member 1' with a high degree of deformability without using additional elastic members as in the embodiment described above, thereby simplifying the structure of the electrode while maintaining its contact characteristics substantially unchanged.

The contact member 1" shown in figure 5 instead is made up of a plurality of elastic members 4" in the form of cantilevers that are restrained e.g. by snapping to the supporting member 2. At the free end of each elastic member 4" contact pads 3" are respectively mounted. This embodiment is characterized by a further simplification of the structure of the electrode, which still maintains its high degree of deformability, and allows to vary in a very simple way the number of contact pads 3" and related elastic members 4" e.g. depending on the position of the electrode on the patient's body and/or on the type of electrocardiogram to be recorded.

Similarly to the two embodiments described above, also in this case may the elastic members 4" be arranged at an angle with respect to the supporting member 2, so as to protrude towards the patient's body in an operative condition.

According to another aspect of the invention, the elastic members 4, 4' and 4" can be made of shape memory materials and be configured so as to be bent or curved towards the patient's body in an operative condition at temperatures proximate or corresponding to the patient's average body temperature. This allows to further improve the contact pressure.

Among the means used to press the electrodes against the patient's body, chest belts are generally preferred, because they allow to exert a higher pressure on the electrodes and also to simultaneously arrange a plurality of electrodes on the patient's body.

The present invention also relates to an electrocardiograph chest belt comprising a plurality of the electrodes described above. As it is known in fact the combination between an electrocardiograph chest belt and a plurality of electrodes allows to facilitate and to speed up the preparation of a patient for the registration of an electrocardiogram.

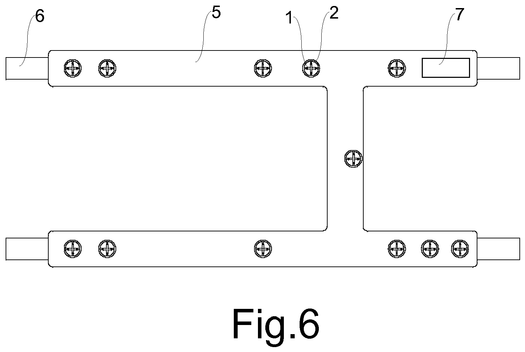

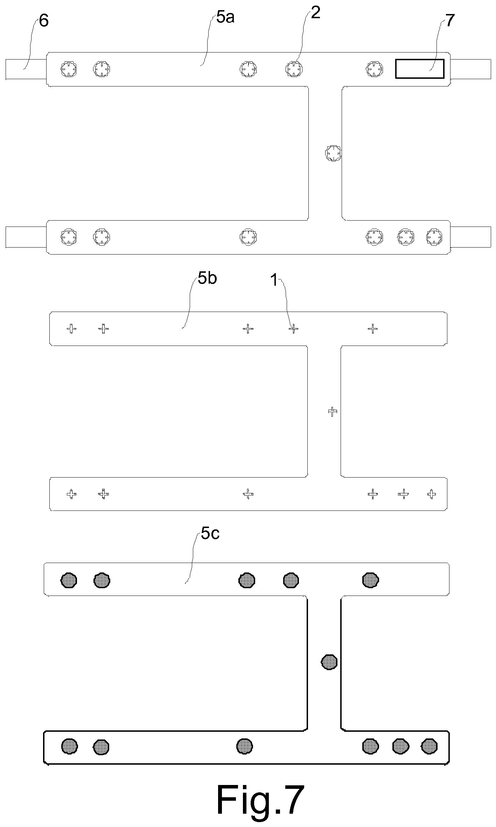

Referring now to figures 6 and 7, which show an electrocardiograph chest belt 5 according to the invention, the electrodes are arranged so that the contact members protrude from an internal side of the chest belt 5, i.e. the side intended to contact the patient's skin. The wires (not shown) connected to the contact pads of the electrodes may be conveniently grouped, restrained to and housed in an external side of the chest belt 5, i.e. the side of the chest belt 5 opposite to the internal one. Alternatively, the wires may be grouped, restrained to and housed in the thickness of the chest belt 5, thus allowing to completely hide and protect them, as well as to prevent a user from coming into contact with these electric components.

The chest belt 5 may also advantageously comprise one or more wireless transmitters 7 connected to the contact pads 3 through the electric wires and capable of transmitting the acquired signals to a remote control unit. This makes it possible to completely eliminate the wires typically used in electrocardiographs for connecting the electrodes and/or the chest belts to the remote control unit, with remarkable advantages in terms of overall size, ease of installation and time of execution of an electrocardiogram.

Figure 6 shows, in particular, a chest belt 5 provided with closing means 6, such as straps or Velcro, and comprising a plurality of electrodes arranged according to a predefined pattern, such a standard twelve-lead pattern. In this embodiment the chest belt 5 comprises a single wireless transmitter 7 arranged near one of the closing means 6.

The chest belt 5 may also advantageously comprise several components to be assembled with each other and so configured to support the various members of the electrodes and their electric components separately.

In the embodiment shown in figure 7, the chest belt 5 comprises two components that can be joined to each other e.g. by Velcro, adhesive members or similar connecting means. The supporting members 2 of the electrodes, the electric wires (not shown) and the transmitter or transmitters 7 are accommodated in an external component 5a of the chest belt 5, i.e. a component intended to face away from the patient's body, while the contact members 1, for example of the type shown in figures 1 to 3, are housed in an internal component 5b of the chest belt 5, i.e. a component intended to contact the patient's body.

This configuration makes it very easy, quick and cheap to replace the contact members of the electrodes once registered an electrocardiogram, as well as when they are worn or damaged, because only the internal component 5b will be removed and replaced by a new one without modifying the arrangement of the supporting members 2, the electric wires and the transmitter 7, which are associated with the external component 5a.

As shown in figure 7, the chest belt 5 may also advantageously comprise an additional component 5c intended to be mounted on the internal component 5b e.g. by Velcro, adhesive members or similar connecting means and to contact the patient's skin in place of the internal component 5b. The additional component 5c comprises a plurality of portions made of a highly conductive material, e.g. silver, gold or a conductive ink, so arranged to match the locations of the electrodes mounted in the internal and external components 5b, 5a. The use of the additional component allows to establish a good electric contact between the electrodes and the patient's skin without establishing a direct contact between the patient's skin and the contact pads. Moreover, it is possible to reuse the same chest belt and electrodes for more than one patient by simply replacing or sterilizing the additional component 5c.

## Claims

1. An electrocardiograph electrode comprising a contact member (1) and a plurality of contact pads (3) arranged on said contact member (1) and suitable to respectively establish an electrical contact with the body of a patient, wherein said contact member (1) is made up of a body comprising a plurality of arms (1a) protruding radially outwards from a central portion (1b), the contact pads (3) being respectively arranged at the ends of each of said arms (1a), **characterized by** further comprising a ring-shaped supporting member (2), the contact member (1) being restrained to said supporting member (2) at the ends of said arms (1a) or at said central portion (1b) by way of a plurality of elastic members (4) in the form of arm members protruding radially inwards from the ring-shaped supporting member (2).

2. An electrode according to claim 1, **characterized in that** the contact member (1) is removably mounted on the supporting member (2).

3. An electrode according to claim 1 or 2, **characterized in that** said elastic members (4) are made of shape memory materials and are configured so as to be bent or curved towards the patient's body in an operative condition at temperatures proximate or corresponding to the patient's average body temperature.

4. An electrode according to any one of claims 1 to 3, **characterized in that** the contact pads (3) are covered by layer of a ion gel.

5. An electrode according to claim 4, **characterized in that** the overall shape of the contact pads (3) covered by the layer of ion gel is convex.

6. An electrode according to any one of claims 1 to 5, **characterized in that** the contact member (1) is concave-shaped and its cavity is arranged so as to face away from the supporting member (2) of the electrocardiograph electrode.

7. An electrocardiograph chest belt (5) comprising one or more electrocardiograph electrodes according to any of claims 1 to 6 and a plurality of electrical wires connected to the contact pads (3) of each one of said electrodes, and wherein said electrical wires are restrained to said chest belt (5).

8. A chest belt (5) according to the previous claim, **characterized by** comprising one or more wireless transmitters (7), said transmitters (7) being respectively connected to each one of the contacts pads (3) arranged on the contact member (1) of each electrocardiograph electrode and suitable to send the data acquired by each electrode to a remote control unit.

9. A chest belt (5) according to claim 7 or 8, **characterized in that** the electrical wires connected to the contact pads (3) are housed in the thickness of the chest belt.

10. A chest belt (5) according to any one of claims 7 to 9, **characterized by** comprising two components (5a, 5b) connectable to one another, the supporting members (2) of the electrodes, connecting wires and a transmitter or transmitters (7) being arranged on an external component (5a) of the chest belt (5) and the contact members (1) being arranged on an internal component (5b) of the chest belt (5).

11. A chest belt (5) according to claim 10, **characterized by** further comprising an additional component (5c) adapted to be mounted on the internal component (5b) and to contact the patient's skin, said additional component (5c) comprising a plurality of portions made of a highly conductive material and so arranged to match the locations of the electrodes mounted in the internal and external components (5b, 5a).

## Patentansprüche

1. Eine Elektrokardiograph-Elektrode, umfassend ein Kontaktelement (1) und eine Mehrzahl von an dem besagten Kontaktelement (1) angeordneten Kontaktstellen (3), welche geeignet sind, jeweils einen elektrischen Kontakt mit dem Körper eines Patienten herzustellen, wobei das besagte Kontaktelement (1) aus einem Körper mit einer Mehrzahl von aus einem zentralen Bereich (1b) radial nach außen vorstehenden Armen (1a) besteht, wobei die Kontaktstellen (3) an den Enden von jeweils einem der besagten Arme (1a) angeordnet sind, gekennzeichnet ferner durch ein ringförmiges Stützelement (2), wobei das Kontaktelement (1) an dem besagten Stützelement (2) über die Enden der besagten Arme (1a) oder über den besagten Bereich (1b) mittels einer Mehrzahl von elastischen Elementen (4) in Form von radial von dem ringförmigen Stützelement (2) nach innen vorstehenden Armelementen gehalten ist.

2. Eine Elektrode gemäß Anspruch 1, **dadurch gekennzeichnet, dass** das Kontaktelement (1) lösbar an dem Stützelement (2) befestigt ist.

3. Eine Elektrode gemäß Anspruch 1 oder 2, **dadurch gekennzeichnet, dass** die besagten, elastischen Elemente (4) aus Werkstoffen mit einem Formgedächtnis bestehen und derart konfiguriert sind, dass sie in einem Betriebszustand bei Temperaturen nahe bei oder entsprechend der mittleren Körpertemperatur des Patienten zu dem Körper des Patienten hin gebogen oder gewölbt sind.

4. Eine Elektrode gemäß einem der Ansprüche 1 bis 3, **dadurch gekennzeichnet, dass** die Kontaktstellen (3) von einer Schicht eines Ionen-Gels bedeckt sind.

5. Eine Elektrode gemäß Anspruch 4, **dadurch gekennzeichnet, dass** die gesamte Form der von einem Ionen-Gel bedeckten Kontaktstellen (3) konvex ist.

6. Eine Elektrode gemäß einem der Ansprüche 1 bis 5, **dadurch gekennzeichnet, dass** das Kontaktelement (1) eine konkave Gestalt aufweist, wobei seine Höhlung derart angeordnet ist, dass sie von dem Stützelement (2) der Elektrode des Elektrokardiographen abgewandt ist.

7. Ein Brustgurt (5) für einen Elektrokardiograph, umfassend eine oder mehrere Elektrokardiograph-Elektroden gemäß einem der Ansprüche 1 bis 6 und eine Mehrzahl von elektrischen Drähten, welche an den Kontaktstellen (3) von jeder der besagten Elektroden angeschlossen sind, wobei die besagten, elektrischen Drähte an dem besagten Brustgurt (5) gehalten sind.

8. Ein Brustgurt (5) gemäß dem vorangehenden Anspruch, **gekennzeichnet durch** einen oder mehrere, drahtlose Sender (7), wobei die besagten Sender (7) jeweils an jeder der an dem Kontaktelement (1) jeder Elektrokardiograph-Elektrode angeordneten Kontaktstellen (3) angeschlossen sind und geeignet sind, die von jeder Elektrode erhaltenen Daten an eine entfernte Steuerungeinheit zu senden.

9. Ein Brustgurt (5) gemäß Anspruch 7 oder 8, **dadurch gekennzeichnet, dass** die an die Kontaktstellen (3) angeschlossenen, elektrischen Drähte in der Schichtdicke des Brustgurtes untergebracht sind.

10. Ein Brustgurt (5) gemäß einem der Ansprüche 7 bis 9, **gekennzeichnet durch** zwei untereinander verbindbare Komponenten (5a, 5b), wobei die Stützelemente (2) der Elektroden, Verbindungsdrähte und ein Sender oder mehrere Sender (7) an einer externen Komponente (5a) des Brustgurtes (5) angeordnet sind, während die Kontaktelemente (1) an einer internen Komponente (5b) des Brustgurtes (5) angeordnet sind.

11. Ein Brustgurt (5) gemäß Anspruch 10, ferner **gekennzeichnet durch** eine zusätzliche Komponente (5c), die geeignet ist, an der internen Komponente (5c) montiert zu werden und mit der Haut des Patienten in Kontakt zu treten, wobei die besagte, zusätzliche Komponente (5c) eine Mehrzahl von Bereichen umfasst, welche aus einem gut leitfähigen Material bestehen und derart angeordnet sind, dass sie mit den Orten der in den internen und externen Komponenten (5b, 5a) montierten Elektroden übereinstimmen.

## Revendications

1. Electrode pour électrocardiographe comprenant un élément de contact (1) et une pluralité de plots de connexion (3) agencés sur ledit élément de contact (1) et appropriés pour établir respectivement un contact électrique avec le corps d'un patient, dans laquelle ledit élément de contact (1) est composé d'un corps comprenant une pluralité de bras (1a) faisant saillie radialement vers l'extérieur à partir d'une partie centrale (1b), les plots de connexion (3) étant respectivement agencés aux extrémités de chacun desdits bras (1a), **caractérisée en ce qu'**elle comprend en outre un élément de support de forme annulaire (2), l'élément de contact (1) étant retenu sur ledit élément de support (2) aux extrémités desdits bras (1a) ou au niveau de ladite partie centrale (1b) au moyen d'une pluralité d'éléments élastiques (4) se présentant sous la forme d'éléments de bras faisant saillie radialement vers l'intérieur à partir de l'élément de support de forme annulaire (2).

2. Electrode selon la revendication 1, **caractérisée en ce que** l'élément de contact (1) est monté, de manière amovible, sur l'élément de support (2).

3. Electrode selon la revendication 1 ou 2, **caractérisée en ce que** lesdits éléments élastiques (4) sont composés de matériaux à mémoire de forme et sont configurés afin d'être pliés ou incurvés vers le corps du patient dans une condition opérationnelle à des températures proches ou correspondant à la température corporelle moyenne du patient.

4. Electrode selon l'une quelconque des revendications 1 à 3, **caractérisée en ce que** les plots de connexion (3) sont recouverts par une couche de gel ionique.

5. Electrode selon la revendication 4, **caractérisée en ce que** la forme globale des plots de connexion (3) recouverts par la couche de gel ionique, est convexe.

6. Electrode selon l'une quelconque des revendications 1 à 5, **caractérisée en ce que** l'élément de contact (1) a une forme concave et sa cavité est agencée afin d'être orientée à l'opposé de l'élément de support (2) de l'électrode pour électrocardiographe.

7. Ceinture thoracique d'électrocardiographe (5) comprenant une ou plusieurs électrodes pour électrocardiographe selon l'une quelconque des revendications 1 à 6 et une pluralité de fils électriques raccordés aux plots de connexion (3) de chacune desdites électrodes, et dans laquelle lesdits fils électriques sont retenus sur ladite ceinture thoracique (5).

8. Ceinture thoracique (5) selon la revendication précédente, **caractérisée en ce qu'**elle comprend un ou plusieurs émetteurs sans fil (7), lesdits émetteurs (7) étant respectivement raccordés à chacun des plots de connexion (3) agencés sur l'élément de contact (1) de chaque électrode pour l'électrocardiographe et appropriés pour envoyer les données acquises par chaque électrode, à une unité de commande à distance.

9. Ceinture thoracique (5) selon la revendication 7 ou 8, **caractérisée en ce que** les fils électriques raccordés aux plots de connexion (3) sont logés dans l'épaisseur de la ceinture thoracique.

10. Ceinture thoracique (5) selon l'une quelconque des revendications 7 à 9, **caractérisée en ce qu'**elle comprend deux composants (5a, 5b) pouvant être raccordés l'un à l'autre, les éléments de support (2) des électrodes, les fils de connexion et un émetteur ou des émetteurs (7) étant agencés sur un composant externe (5a) de la ceinture thoracique (5) et les éléments de contact (1) étant agencés sur un composant interne (5b) de la ceinture thoracique (5).

11. Ceinture thoracique (5) selon la revendication 10, **caractérisée en ce qu'**elle comprend en outre un composant supplémentaire (5c) adapté pour être monté sur le composant interne (5b) et pour être en contact avec la peau du patient, ledit composant supplémentaire (5c) comprenant une pluralité de parties réalisées avec un matériau très conducteur et agencé afin de correspondre aux emplacements des électrodes montées dans les composants interne et externe (5b, 5a).
